# EUROPÄISCHE PATENTSCHRIFT

(11) **EP 2 050 477 B1**
(45) Veröffentlichungstag und Bekanntmachung des Hinweises auf die Patenterteilung: **26.12.2018**
(21) Anmeldenummer: 07118924.5
(22) Anmeldetag: 19.10.2007
(51) Int. Cl.: A61M 5/315, A61M 5/31, A61M 5/24

(54) **Ampulleneinheit mit Adapter**
Ampoule unit with adapter
Unité d'ampoule dotée d'un adaptateur

(43) Veröffentlichungstag der Anmeldung: 22.04.2009
(73) Patentinhaber: F. Hoffmann-La Roche AG, 4070 Basel (CH); Roche Diabetes Care GmbH, 68305 Mannheim (DE)
(72) Erfinder: Rufer, Thomas, 3072 Ostermundigen (CH); Burkhalter, Rolf, 3600 Thun (CH); Heiniger, Hanspeter, 4932 Lotzwil (CH); Stoller, Hanspeter, 3013 Bern (CH)
(74) Vertreter: Rentsch Partner AG

(56) Entgegenhaltungen:
- WO-A-2004/016303
- GB-A- 2 058 228
- US-A- 4 189 065
- US-A- 5 059 179
- US-A- 5 618 273
- US-A1- 2002 113 088

## Beschreibung

Die Erfindung betrifft eine Ampulleneinheit zum Einbau in ein Verabreichungsgerät, bevorzugt ein Injektionsgerät oder weniger bevorzugt eine Injektionsspritze, wobei die Ampulleneinheit wenigsten eine Ampulle und einen Adapter umfasst, und die Ampulle mit dem Adapter, der ein Gewinde aufweisen kann, anders als über dieses Gewinde verdreh- und verschiebesicher verbindbar ist.

Im Stand der Technik sind zahlreiche Verabreichungsgeräte zur Ausschüttung von Fluiden, beispielsweise Medikamente wie Insulin, Hormonpräparate oder andere medizinische oder kosmetische Stoffe bekannt. Bei den allermeisten dieser Geräte, insbesondere wenn die Ausschüttung über eine Nadel am distalen Ende der Ampulle oder des Gerätes bewirkt wird, wird das Fluid durch einen linear geführten Kolben ausgeschüttet. Besonders bei kleinen Basalraten ergeben sich dabei Slipstick-Probleme dadurch, dass der die Ausschüttung bewirkende Stopfen oder Kolben, der durch beispielsweise eine Kolbenstange angetrieben wird, gleichzeitig auch die Dichtfunktion für die Ampullenrückseite übernimmt und zu diesem Zweck bevorzugt aus einem gummiartigen Werkstoff geformt ist. Bei einem rein linear gleitenden Vortrieb dieses Stopfens oder Kolbens kommt es zwischen diesem und der Ampulleninnenwand immer wieder zu einem Wechsel zwischen Haftreibung und Gleitreibung. Das heißt aber, dass es teils zu verzögerten, sprunghaften Vortriebsbewegungen des Kolbens kommt, wodurch eine gleichmäßige Abgabe des Fluids aus der Ampulle nicht gewährleistet ist.

Dies ist besonders dann von Nachteil, wenn aus einer Ampulle mehrere oder zahlreiche kleinste Abgabemengen nacheinander, das heißt, in einem vorzugsweise definierten, einstellbaren zeitlichen Abstand, abgegeben werden sollen. Wird bei einer solchen Ausschüttung der Kolben durch die Haftreibung zurückgehalten, kommt es zu einer zu kleinen Ausschüttung, trotz der linearen Vorwärtsbewegung der Kolbenstange. Bei der nächsten oder einer späteren Ausschüttung kann die durch die Haftreibung bedingte Rückhaltung des Kolbens überwunden werden, wodurch der Kolben insgesamt jetzt eine größere Distanz als die geplante zurücklegt, mit dem Ergebnis, dass die diesmal ausgeschüttete Dosis zu groß ist. Der Patient oder Nutzer kann folglich bei kleinen Abgabemengen des fluiden Stoffes bis zur völligen Entleerung der Ampulle mehrfach unter- oder überversorgt werden. Dabei wird dieses Problem offensichtlich größer, je kleiner die pro Anwendung abzugebende Fluidmenge ist, im Extremfall kann dies zu einem Wechsel zwischen der Abgabemenge Null (bei gänzlicher Rückhaltung des Kolbens durch die Haftreibung) und einer vielfachen Abgabemenge in einem nächsten Schritt führen.

Die US 2003/0167039 A1 beschreibt eine Pumpe zum Ausschütten eines Fluids aus einem Reservoir. Die Pumpe besteht aus einem ersten Teil zur Aufnahme des Reservoirs mit Kolbeneinheit, beispielsweise einer Ampulle, und einem zweiten Teil, zur Aufnahme des Motors und der Kolbenvortriebseinrichtung. Beide Teile sind nebeneinander angeordnet, wodurch die Baulänge der Pumpe reduziert wird. Auf die Rückseite der Kolbeneinheit wirkt eine starre, fest mit einer Mutter verbundene Platte, die auf einer drehangetriebenen Gewindestange sitzt, die mit einem Motor verbunden ist. Dreht sich der Motor, so bewegt sich die Mutter in die Vortriebsrichtung und schiebt über die Platte die Kolbeneinheit in einer Linearbewegung in Richtung der Fluidausschüttung. Alternativ kann die Gewindestange über ein Zahnrad direkt mit der Kolbenstange verbunden sein. Die Kolbenstange steht in einer Gewindeverbindung mit dem Kolben und bewegt diesen linear in die Vortriebsrichtung.

Aus der EP 1 752 172 A1 ist eine Antriebsvorrichtung für eine Infusionspumpe bekannt. In Figur 4 wird, wie in Paragraph 0030 beschrieben, eine Ampulle mit einem Innengewinde gezeigt, in das ein Kolben mit einem Außengewinde eingreift, der dadurch um seine Drehachse drehend in die Ampulle eingeschraubt wird und dadurch das in der Ampulle enthaltenen Fluid ausschüttet. Um die Ampulle der EP 1 752 172 A1 vollständig entleeren zu können, muss das Innengewinde über die gesamte Innenlänge der Ampulle ausgebildet sein.

Aus der US 2002/0113088 A1 ist eine Spritze bekannt, mit einem Spritzenkörper mit einem Aussengewinde, auf den eine Mutter mit einem ersten Innengewinde aufgeschraubt werden kann. Die Mutter weist ein zweites Innengewinde auf, in das zur Ausschüttung des Spritzeninhalts eine Kolbenstange eingeschraubt und in die Ausschüttrichtung vorgeschraubt werden kann. Die WO 2004/016303 A1 beschreibt eine Spritze, bestehend aus einer Ampulle mit einem ausgeprägten Bund am proximalen Ende und einem Adapter, der auf den Bund aufgeschoben oder aufgeklippt werden kann. Der Adapter hat ein Innengewinde, in den eine Kolbenstange eingeschraubt werden kann, wodurch das Produkt aus der Ampulle ausgeschüttet wird. Aus der US 4,189,065 ist eine Abgabevorrichtung bekannt mit einer Ampulle mit Bund und einem Adapter, der auf den Ampullenbund aufgeschoben werden kann. Mit dem Adapter kann ein Gewindeelement aufgesteckt werden, das es ermöglicht, dass zur Ausschüttung des Produkts aus der Ampulle eine Kolbeneinheit mit einem Aussengewinde an der Kolbenstange schraubend in die Ampulle eingeführt werden kann.

US 5059179 zeigt eine manuell zu bedienende Einwegspritze für medizinische Flüssigkeiten, die derart ausgestaltet ist, dass sie nach einmaligem Befüllen aus dem leeren Zustand und anschliessender funktionsgemässer Entleerung kein zweites Mal befüllt werden kann. Während des Entleerens der Spritze wird die Kolbenstange irreversibel vom Kolben angetrennt. Bei einer Ausführungsform sind Kolbenstange und Kolben über ein erstes Gewinde verbunden Die mit einem zweiten Aussengewinde versehene Kolbenstange wird beim Herausziehen durch ein Gegengewinde einer fest am proximalen Ende des Zylinders montierten Mutter in eine erste Drehrichtung rotiert. Das erste Gewinde am Kolben ist gegenläufig zum zweiten Gewinde, so dass der Kolben beim Herausziehen rotierend mitgeschleppt wird. Wird anschliessend die Kolbenstange wieder in die Spritze hineingedrückt, um die Spritze zu entleeren, dreht die Kolbenstange in die entgegen gesetzte Richtung. Aufgrund der Haftreibung mit dem Spritzenzylinder dreht der Kolben nicht mit, und stattdessen wird die Kolbenstange aus dem ersten Gewinde des Kolbens geschraubt. Wenn der Kolben sich am distalen Ende des Zylinders befindet, sind Kolben und Kolbenstange vollständig und dauerhaft entkoppelt. Wird beim Versuch einer erneuten Befüllung der Spritze die Kolbenstange wieder herausgezogen, verbleibt der Kolben am distalen Ende des Zylinders, und eine erneute Befüllung der Spritze wird verunmöglicht.

Es ist Aufgabe der Erfindung eine Ampulleneinheit für Verabreichungsgeräte bereitzustellen, die es ermöglicht, dass bei der mehrfachen Abgabe von gleichen, kleinen Menge aus einer Ampulle, die jeweils abgegebene Menge immer annhähernd gleich groß ist.

Erfindungsgemäß wird diese Aufgabe durch eine Ampulleneinheit nach Anspruch 1 gelöst.

Die erfindungsgemäße Ampulleneinheit nach Anspruch 1 umfasst eine Ampulle, bevorzugt eine handelsübliche Ampulle für ein Verabreichungsgerät mit einem proximalen und einem distalen Ende. Dabei soll als proximal immer das Ende der Ampulle bezeichnet werden, dass beim Einsatz der Ampulle in dem Verabreichungsgerät weg von der Abgabeseite, die beispielsweise mit einer Injektionsnadel oder Injektionsdüse verbunden oder verbindbar ist, über die das in der Ampulle enthaltenen Fluid in einen menschlichen oder tierischen Körper abgegeben wird, weist. Als distal wird entsprechend die der proximalen Seite gegenüberliegende Seite der Ampulle bezeichnet, die zum Einsatz der Ampulleneinheit beispielsweise mit einem Nadelträger, einer Nadel oder Düse verbindbar ist, oder bereits im Lieferzustand mit einem dieser Elemente verbunden ist.

Die Ampulle kann als Leerampulle bevorratet werden, die erst kurz vor dem Einsatz mit dem Fluid befüllt wird, ebenso kann sie aber bereits befüllt an den Nutzer geliefert werden, was insbesondere bei einer Abgabe an einen Selbstverabreicher die bevorzugte Art der Bereitstellung ist.

Die Ampulleneinheit umfasst neben der Ampulle einen Adapter, der mit der Ampulle verdreh- und verschiebesicher verbunden ist und der die Ampulle wenigstens teilweise umgibt und/oder teilweise in die Ampulle eingreift. Der Adapter kann aus dem gleichen Material wie die Ampulle bestehen, beispielsweise Glas oder Kunststoff, er kann aber auch aus einem anderen Material, beispielsweise Metall oder Kunststoff bestehen. Er wird in der Regel separat zur Ampulle hergestellt und erst zu einem späteren Zeitpunkt mit der Ampulle verbunden. Diese Verbindung kann durch Formschluss, Verklebung, Verschweißung oder andere Verbindungen hergestellt werden, solange gesichert ist, dass die Ampulle sich zum Adapter relativ weder in Längsrichtung noch in Umfangsrichtung bewegen kann.

Ein Vorteil der separaten Herstellung von Ampulle und Adapter ist, dass die Ampullenkörper in großer Stückzahl hergestellt oder eingekauft werden können, und die Adapter für beispielsweise verschiedene Abgabedosen mit Gewinden unterschiedlicher Steigung in kleinerer Stückzahl hergestellt werden können. Der notwendige zweite Schritt des Zusammenfügens von Ampulle und Adapter könnte dann erst unmittelbar vor der Befüllung der Ampullen, bzw. bei unbefüllten Ampullen unmittelbar vor deren Auslieferung durchgeführt werden.

Der Adapter weist ein Gewinde auf, das mit dem Gegengewinde eines in der Ampulle wirkenden Verdrängungskörpers, der die Ausschüttung des Produkts aus der Ampulle durch die distale Öffnung der Ampulle bewirkt, in einem Gewindeeingriff ist.

Der Adapter weist bevorzugt wenigstens an seinem proximalen Ende ein Gewinde auf. Prinzipiell kann eine einfache Mutter, die auf dem proximalen Ende der Ampulle befestigt oder befestigbar ist, als Adapter dienen. Die Ampulle ist in diesem Fall bevorzugt ein handelsüblicher Ampullenkörper. Der Adapter kann die Ampulle aber auch teilweise umgeben, bzw. im proximalen Bereich in die Ampulle hineinragen. Außerdem kann er auch die Ampulle ganz umgeben in dem Sinne, dass die Ampulle in dem Adapter aufnehmbar ist. Der Adapter kann schließlich auch formschlüssig direkt auf die Ampulle aufgebracht, beispielsweise aufgeschrumpft oder aufgespritzt sein. Die Verbindung zwischen Adapter und Ampulle kann andererseits auch lösbar ausgebildet sein, das heißt, die Ampulle kann nachträglich in den Adapter eingesetzt und nach der Leerung aus dem Adapter entfernt werden. Dazu können beispielsweise außen am Ampullenkörper Rastelemente angeformt sein, die mit Rastgegenelementen am Adapter für eine verdreh- und verschiebesichere Verbindung zwischen Adapter und Ampulle sorgen. Wichtig ist in jedem Fall, dass der Adapter sich relativ zur Ampulle nicht bewegen kann, sondern dass die beiden Teile jede Linearbewegung und jede Drehbewegung nur als Einheit durchführen können.

Der Adapter kann auch direkt von der Innenseite des Gehäuses als Teil des Gehäuses des Verabreichungsgeräts gebildet sein oder als separates Teil fest mit dem Gehäuse verbunden sein.

Im Folgenden werden bevorzugte Weiterbildungen der Ampulleneinheiten nach Anspruch 1 beschrieben.

Die Ampulleneinheit umfasst zur Abgabe des Fluidprodukts eine Kolbeneinheit. Über diese Kolbeneinheit wird dann Druck auf das Fluidprodukt ausgeübt, um beispielsweise seine Ausschüttung aus der Ampulle und seine Injektion in ein Gewebe zu bewirken. Dazu weist die Kolbeneinheit, die beispielsweise aus einem Kolben und einer Kolbenstange besteht, am Kolben oder an der Kolbenstange ein Gewinde auf, das ein Gegengewinde zu dem Gewinde des Adapters bildet. Beide Gewinde sind in einen Gewindeeingriff gebracht, das heißt, die Kolbeneinheit ist in oder auf das beispielsweise proximale Ende des Adapters ein- bzw. aufgeschraubt. Dabei ist der Kolben in das proximale Ende der Ampulle eingeführt und wird bei einer weiteren gleichgerichteten Drehung schraubend in die Ampulle in die Vortriebsrichtung bewegt.

Der Kolben der Kolbeneinheit kann aus einem Material gefertigt sein, dass elastisch genug ist, dass es die Ampulle in proximale Richtung abdichtet und gleichzeitig einen möglichst geringen Gleitreibungswiderstand mit dem Material der Ampulleninnenseite aufweist. Alternativ oder zusätzlich kann auf der distalen Kolbenoberfläche eine Dichtung befestigt sein. Zur Verringerung des Gleitreibungswiderstands kann der Kolben an seinem Umfang eine konkave Form haben, wodurch der Berührungsbereich zwischen Kolben und Ampulleninnenwand auf das in Vortriebsrichtung vorlaufende und nachlaufende Ende des Kolbens begrenzt wird. Der Kolben kann aber auch aus einem harten Zylinderkern bestehen, an dem eine, zwei oder mehrere Dichtringe angespritzt sind. Das Material der Dichtringe kann so gewählt werden, dass hohe Dichtigkeit und geringer Reibungswiderstand erreicht werden. Zur Reduzierung des Haft- und/oder Gleitreibungswiderstandes können die Ampulleninnenseite und/oder die Kolbenaußenseite bzw. die Dichtungen mit einem die Reibung reduzierenden Mittel beschichtet sein.

Bei dem Gewinde des Adapters kann es sich grundsätzlich um ein Innen- oder Außengewinde handeln. Bevorzug ist das Gewinde an der Außenwand des Adapters gebildet. Als Außenwand wird in diesem Zusammenhang die Seite des Adapters bezeichnet, die von der Ampulle weg nach außen weist. Wenn von einem Innengewinde des Adapters die Rede ist, so ist darunter ein Gewinde zu verstehen, dessen Zähne vom Adapter in Richtung zur Drehachse der Ampulle abragen, unabhängig davon, ob sie, wie beispielsweise beim Hineinragen des Adapters in das proximale Ende der Ampulle, an dessen Außenseite angeformt sind. In beiden Fällen ist die Kolbeneinheit so geformt, das einerseits das Gegengewinde mit dem Gewinde des Adapters im Gewindeeingriff steht, während gleichzeitig ein zentraler Teil der Kolbeneinheit die Ausschüttung des Fluidprodukts aus der Ampulle bewirkt.

Weniger bevorzugt handelt es sich bei dem Gewinde des Adapters um ein Innengewinde, was bedeutet, dass der Adapter von der proximalen Seite in die Ampulle hineinreicht und die Kolbeneinheit von der proximalen Seite der Ampulleneinheit in das Adaptergewinde eingeschraubt werde kann. Da das Gewinde bei dieser Ausführungsform bevorzugt nur in einem proximalen Endabschnitt der Ampulle ausgebildet ist, kann die Ampulle in diesem Fall in proximale Richtung durch ein Dichtelement, vorzugsweise eine Dichtungsscheibe abgeschlossen sein, die beispielsweise direkt an einem senkrecht von der Ampullenwand abstehenden waggerechten Adapterende anliegt. Bevorzugt weist die Dichtungsscheibe eine axiale Dicke auf und kann mit der Kolbeneinheit durch beispielsweise Formschluss so verbunden werden, dass sie sich mit dem in die Ampulle hineinschraubende Kolben mitdreht und weiterhin als Dichtelement dient. Die Dichtscheibe kann im Sinne der Erfindung auch den Kolben der Kolbeneinheit bilden. In das proximale Ende hineinreichen bedeutet hier, dass der Adapter defacto an der Außenseite des Adapters anliegt und dass sein proximales Ende um das proximale Ende der Ampulle umgebogen ist, so dass das umgebogene Ende des Adapters in das proximale Ende der Ampulle reicht.

Gewinde und Gegengewinde weisen im Regelfall eine Gewindesteigung von 0 mm/U bis 2 mm/U auf, bevorzugt liegt sie zwischen 0,5 mm/U und 2 mm/U. Weniger bevorzugt kann die Gewindesteigung auch im Bereich ≤ 1 mm/U liegen, in Einzelfällen mehr als 2 mm/U betragen.

Die Kolbeneinheit, die einstückig sein kann, wird bei bestehendem Gewindeeingriff zwischen Kolbeneinheit und Adapter zur Ausschüttung des Fluidprodukts in die Ampulleneinheit geschraubt. Das heißt, der die Ausschüttung bewirkende Teil der Kolbeneinheit, das heißt deren vorderes Ende im Fall der Innengewindelösung oder im Falle der Außengewindelösung der in der Ampulle geführte Teil, wird, sich um seine Längsachse drehend, in die gegen ein Mitdrehen gesicherte Ampulleneinheit hineingeschraubt. Diese kombinierte rotative und lineare Bewegung der Kolbeneinheit führt dazu, dass das die Ausschüttung bewirkende vordere Ende der Kolbeneinheit einen spiralförmigen Weg zurücklegt, der größer ist, als der Weg einer rein linearen Bewegung, wie im Stand der Technik üblich. Ein längerer Weg ermöglicht aber sicherer die Überwindung der Haftreibung zwischen Ampulleninnnenfläche und Kolbeneinheitaußenfläche und den Übergang in die Gleitreibung.

Dadurch, dass sich die Kolbeneinheit in der Ampulle nicht ausschließlich linear bewegen lässt, kann beispielsweise ein Freeflow effizient unterbunden werden, bei gleichzeitiger Minimierung des Slipsticks. Um die Schraubbewegung der Kolbeneinheit zu bewirken, genügt ein einfacher rotativer Antrieb für die Kolbeneinheit. Der Gewindeeingriff zwischen Adapter und Kolbeneinheit führt bei durch den Adapter fest gehaltener Ampulle zu einer Verkürzung der Dehnungskette des Systems gegenüber herkömmlichen Lösungen, einer besser definierten Kolbenposition in der Ampulle und verringert zudem das Staubolusvolumen und die Verzögerungszeiten bei der Ausschüttung. Dies führt dazu, dass speziell bei der wiederholten Abgabe kleiner oder kleinster Fluidmengen bei jeder Ausschüttung mit großer Sicherheit immer die gleiche Menge an Fluid aus der Ampulle ausgeschüttet wird. Dadurch wird der Anwender zuverlässig reproduzierbar pro Anwendung immer mit der gleichen Fluidmenge, beispielsweise einem Medikament versorgt, was eine Beschränkung der abzugebenden Menge pro Ausschüttung auf das notwendige Minimum erlaubt.

Wie beschrieben kann der Adapter wenigstens an seinem proximalen Ende mit einem Gewinde versehen sein. Die Ausbildung des Gewindes ist aber insbesondere im Falle der Außengewindelösung nicht auf das proximale Ende beschränkt, vielmehr kann die Adapteraußenseite insgesamt als Gewinde geformt sein. Gleiches gilt natürlich wenn der Adapter ein Innengewinde aufweist, das mit einem Außengewinde der Kolbeneinheit zusammenwirkt. Auch hier muss beispielsweise nicht die gesamte Länge des in die Ampulle hineinreichenden Adapterteils als Gewinde ausgebildet sein.

Die Kolbeneinheit kann durch einen Kolben alleine gebildet werden oder durch eine Kombination aus Kolben und Kolbenstange. Besteht die Kolbeneinheit aus Kolben und Kolbenstange, so kann das mit dem Gewinde des Adapters in Eingriff bringbare Gegengewinde entweder am Kolben oder an der Kolbenstange gebildet sein. Der Kolben und die Kolbenstange können einstückig geformt sein. Dabei können sie beispielsweise aus Kunststoff bestehen und mittels eines Spritzgussverfahrens, beispielsweise dem Zweikomponenten-Spritzguss, hergestellt sein. Kolben und Kolbenstange können aber auch getrennt Teile sein, die miteinander unlösbar, beispielsweise mittels einer Klebe- oder Schweißverbindung, verbunden sind. Schließlich kann der Kolben mit der Kolbenstange auch lösbar verbunden sein, wobei die Verbindung eine Schraub- oder Steckverbindung sein kann. In diesem Fall muss aber gewährleistet sein, dass sich Kolben und Kolbenstange immer gemeinsam um ihre Rotationsachse drehen, wenn die Kolbeneinheit in die Ampulleneinheit geschraubt wird. Das heißt, der Kolben darf sich zur Kolbenstange im verbundenen Zustand nicht relativ bewegen, wenn die Gesamtheit von Kolben und Kolbenstange in die Ampulleneinheit geschraubt wird. Das vorderste Ende des Kolbens kann eine Dichtung bilden, die bei der Herstellung am Kolben angeformt oder verdrehsicher mit dem Kolben verbunden, beispielsweise verschweißt, ist.

Die Kolbeneinheit kann aber auch aus dem Kolben, einer mit dem Kolben fest verbundenen Hülse, die das Gewinde trägt, und einem Antriebselement gebildet sein. Die Hülse weist in Längsrichtung eine Aussparung auf, beispielsweise ein quadratisches Sackloch auf, in welches das Antriebselement, in diesem Fall ein entsprechend ausgebildeter Vierkantstab, formschlüssig eingreift. Wird jetzt das Antriebselement, beispielsweise über ein mit seinem proximalen Ende verbundenes Zahnrad angetrieben, drehen sich automatisch die Hülse und der Kolben mit und werden durch die Gewindeverbindung zwischen Hülse und Adapter in Richtung der Ausschüttung geschraubt.

Bei dem Adapter kann es sich um ein Wegwerfgerät handeln, das nach völliger Entleerung der Ampulle entsorgt wird oder um eine wieder verwendbare Einheit, aus der eine leere Ampulle entnommen und eine neue volle Ampulle eingesetzt werden kann. Dadurch können beide Teile unabhängig voneinander entsorgt und recycelt werden.

Die vorbesprochenen Ampulleneinheiten können sowohl in stationären, als auch mobilen Verabreichungsgeräten Verwendung finden.

Nachfolgend werden Ausführungsbeispiele der Erfindung anhand von Figuren erläutert. An den Ausführungsbeispielen offenbar werdende Merkmale bilden je einzeln und in jeder Merkmalskombination die Gegenstände der Ansprüche und auch die vorstehend beschriebenen Ausgestaltungen vorteilhaft weiter. Es zeigen:
Figur 1: Ampulleneinheit mit Adapter, Innengewinde
Figur 2: Ampulleneinheit mit Mutter als Adapter
Figur 3: Ampulleneinheit mit Adapter, Außengewinde
Figur 4. Ampulleneinheit, Adapter als Teil des Gehäuses

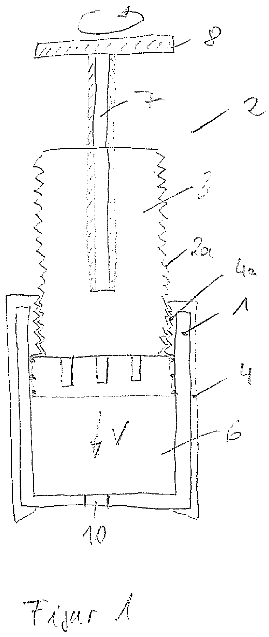

In Figur 1 ist eine Ampulle 1 zu sehen, die in einem Adapter 4 aufgenommen und mit einer Kolbeneinheit 2 verbunden ist. Die Ampulle 1 weist eine distale Öffnung 10 zur Abgabe des Ampulleninhalts aus der Fluidkammer 6 und eine proximale Öffnung, zur Aufnahme und Führung einer Verdrängungseinheit, hier die Kolbeneinheit 2 auf. In das proximale Ende der Ampulle 1 reicht ein Abschnitt des Adapters 4 mit einem an diesem Abschnitt des Adapters 4 gebildeten Gewinde 4a, im Sinne dieser Beschreibung einem Innengewinde, hinein. In diesem ersten Ausführungsbeispiel reicht das Gewinde 4a über den gesamten in der Ampulle 1 liegenden Abschnitt bis zu seinem proximalsten Ende. Das Gewinde 4a kann aber auch nur an einem Teil des in die Ampulle 1 reichenden Adapterabschnitts angeformt sein, beispielsweise weiter in die distale Richtung verschoben. Wichtig ist, dass der Adapter 4 die Ampulle verdreh- und verschiebesicher hält. Das heißt, der Adapter 4 bildet beispielsweise eine weitestgehend starre Hülse, in die die Ampulle 1 eingeschoben werden kann, mit einem distalen Ende, das das distale Ende der Ampulle 1 umgreift, und einem proximalen Ende, das das proximale Ende der Ampulle 1 umgreift. Im gezeigten Ausführungsbeispiel kann die Ampulle 1 beispielsweise von der distalen Seite in den Adapter 4 eingeführt werden. Dabei weist der Adapter 4 an seinem distalen Ende beispielsweise keine geschlossenen Zylinderform auf, sondern Elemente, die sich beim Einführen der Ampulle 1 elastisch nach außen biegen und bei vollständig eingeführter Ampulle 1, das heißt, wenn das proximale äußere Ende der Ampulle 1 am proximalen inneren Ende des Adapters 4 anliegt, in ihre Ausgangslage zurückfedern und so die Ampulle 1 verschiebesicher halten. Das proximale innere Ende des Adapters wird im gezeigten Ausführungsbeispiel durch das proximale Ende des Hohlraumes definiert, den der Adapter 4 an seinem proximalen Ende durch das Umschlagen um das proximale Ampullenende bildet.

Zum Ausschütten des Inhalts der Fluidkammer 6 ist in Figur 1 eine mit dem Adapter 4 zusammenwirkende Kolbeneinheit 2 dargestellt. Diese Kolbeneinheit 2 besteht aus einem Zentralkolben 2b, der einen Außendurchmesser aufweist, der im Wesentlichen dem Innendurchmesser der Ampulle 1 entspricht. An seinem vordersten, distalen Ende weist der Zentralkolben eine Dichtung 5 auf, die den Inhalt der Ampulle 1 daran hindert, bei der Ausschüttung nach hinten aus der Ampulle 1 auszutreten. Diese Dichtung 5 kann fest mit dem Zentralkolben 2b verbunden sein, bevorzugt dient sie aber bereits bei der Bereitstellung der Ampulle 1 als proximale Abdichtung und wird durch die Kolbeneinheit 2 bei der Ausschüttung in die distale Richtung mitgenommen.

Zur Ausschüttung ist die Kolbeneinheit 2 mit einem Antrieb 7, 8 verbunden, der die Kolbeneinheit 2 in Drehung um ihre Drehachse versetzt, wodurch sich die Kolbeneinheit 2 in der Gewindeverbindung 2a, 4a in die Ampulle 1 schraubt und die Ausschüttung des Inhalts der Ampulle 1 durch die Öffnung 10 bewirkt. Eine rein lineare Bewegung des Zentralkolbens 2b in die Ausschüttrichtung V ist nicht möglich. Nur durch eine Drehung der Kolbeneinheit 2 kann der Zentralkolben 2b über das Gewinde 4a und das Gegengewinde 2a in die Ampulle 1 geschraubt werden. Um in die Vortriebsrichtung V eine Distanz x, die einer Ausschüttungsmenge y entspricht zurückzulegen, muss der sich in die Ampulle 1 schraubende Zentralkolben 2b daher einen größeren Laufweg zurücklegen, als dies bei einer rein linearen Bewegung der Fall wäre. Dies führt vorteilhafter Weise dazu, dass eine Haftreibung zwischen Ampulleninnenwand und Kolbenaußenseite sicher in eine Gleitreibung während der Bewegung des Kolbens überführt wird. Dadurch wird auch bei der wiederholten Abgabe kleiner oder kleinster Mengen von Fluid die vorgegebene Ausschüttmenge immer mit großer Zuverlässigkeit erreicht. Eine Verfälschung der einzelnen Ausschüttmenge durch Slipstick-Probleme oder eine lange Dehnungskette des Systems wird weitestgehend ausgeschlossen.

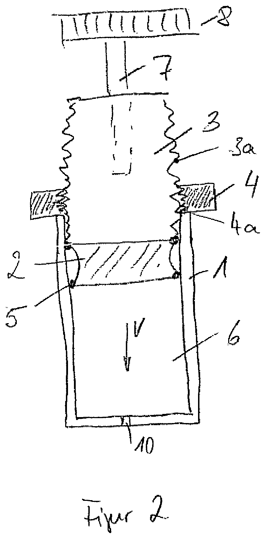

Figur 2 zeigt ein zweites Ausführungsbeispiel der Ampulleneinheit. Mit der Ampulle 1 ist ein Adapter 4, in Form einer Mutter mit Innengewinde fest verbunden. Fest heißt in diesem Zusammenhang, dass sich die Mutter bzw. der Adapter 4 relativ zur Ampulle 1 weder linear noch rotativ bewegen kann. Dies kann durch eine geeignete Klebe- oder Schweißverbindung erreicht werden, ebenso gut aber auch durch eine entsprechende Ausformung des Ampullenendes, in das der Adapter 4 formschlüssig und gesichert gegen jegliche Relativbewegung zur Ampulleneinheit 1 gehalten wird.

Der Kolben 2 ist im Ausführungsbeispiel an seiner Außenseite konkav geformt und weist an dem oberen und unteren Ende umlaufend je eine Dichtung 5 auf. Er kann als unabhängiges Einzelteil geformt sein und bereits im Auslieferzustand der Ampulle 1 als rückwärtige Dichtung dienen. In diesem Fall weist er an seiner proximalen, das heißt, der Vortriebsrichtung V entgegengesetzten Seite, Verbindungselemente auf, mit der er mit einer Kolbenstange 3 verdrehsicher verbunden werden kann. Verdrehsicher soll heißen, dass der Kolben 2 im verbundenen Zustand mit der Kolbenstange 3 jeder Drehbewegung der Kolbenstange mitmacht, zumindest jede Drehbewegung, mit der sich der Kolben 2 in distale Richtung in die Ampulle hineinschraubt und Fluid aus der Fluidkammer 10 durch die Öffnung 10 verdrängt.

Die Kolbenstange 3 weist ein Außengewinde 3a auf, das ein Gegengewinde zum Innengewinde des Adapters 4 ist. Das heißt, die Gewindestange 3 kann sich mit ihrem Gewinde 3a in das Gewinde 4a des Adapters 4 drehen und schraubt sich dadurch in Vortriebsrichtung V in die Ampulle 1 hinein. Die Kolbenstange 3 kann direkt oder über einen nicht gezeigten Stutzen über ein Antriebselement 7 mit einem Zahnrad 8 verbunden sein, dass von einem Motor oder händisch angetrieben wird und dadurch den Kolben 2 in die Vortriebsrichtung V schraubt.

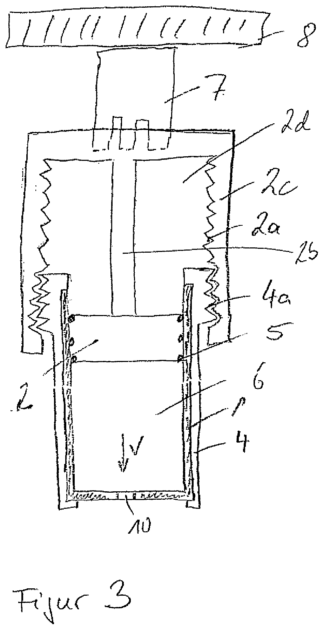

Ein drittes Ausführungsbeispiel zeigt Figur 3. Hier ist die Ampulle 1 in einem Adapter 4 gehalten, der sie teilweise umgibt, so dass sich der Adapter 4 relativ zur Ampulle 1 weder linear noch rotativ bewegen kann. Dazu kann der Adapter 4 mit der Ampulle 1 formschlüssig verbunden, verklebt, verschweiß oder verschrumpft sein. Wichtig ist einzig und allein die weitestgehend starre Verbindung der beiden Teile, die keine Relativbewegung zwischen Ampulle 1 und Adapter 4 zulässt.

Der Adapter 4 weist in seinem hinteren, proximalen Bereich ein Außengewinde im Sinne der Beschreibung auf. Mit diesem in Gewindeeingriff steht eine Kolbeneinheit, die wie die Kolbeneinheit der Figur 1 ausgebildet ist und für die das dort gesagte entsprechend gilt.

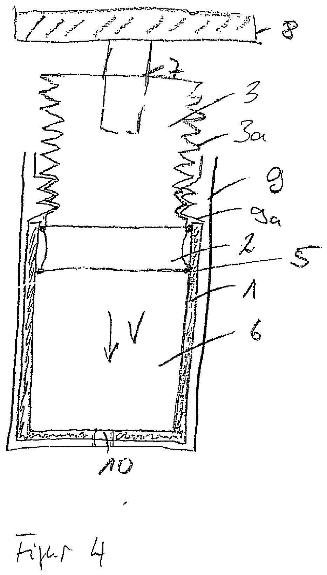

Ein weiteres Ausführungsbeispiel der Erfindung zeigt schließlich die Figur 4. Der erfindungsgemäße Adapter 4 wird hier durch ein Gehäuse 9 gebildet. Dieses Gehäuse 9 kann beispielsweise das Gehäuse des Verabreichungsgerätes sein, oder das Gehäuse einer Untereinheit des Verabreichungsgerätes, die als Gesamtheit in das Verabreichungsgerät eingesetzt wird. Das an der Innenseite der Gehäusewand angeformte Gewinde 9a liegt oberhalb einer in das Gehäuse eingesetzten Ampulle 1. Die Ampulle 1 ist in dem Gehäuse 9 verdreh- und verschiebesichersicher gehalten. Mit dem Innengewinde 9a des Gehäuses 9 ist das Außengewinde 2a einer Kolbeneinheit 2 in Gewindeeingriff. Durch Drehung der Kolbeneinheit 2 um ihre Drehachse, wird die Kolbeneinheit 2 in Vortriebsrichtung V in die Ampulle hineingeschraubt und bewirkt eine Ausschüttung aus der Fluidkammer 6 durch die Öffnung 10. Zur detaillierten Beschreibung wird auf Figur 2 verwiesen. Das dort zur Zusammenwirkung von Adapter 4 und Kolbenstange 3 gesagt gilt ebenso für das Ausführungsbeispiel der Figur 4.

### Bezugszeichenliste

- 1: Ampulle
- 1a: Ampullengewinde
- 2: Kolben / Kolbeneinheit
- 2a: Kolbengewinde
- 2b: Zentralkolben
- 2c: Kolbenhülse
- 2d: Schlitz
- 3: Kolbenstange
- 3a: Kolbenstangengewinde
- 4: Adapter
- 4a: Adaptergewinde
- 5: Dichtung
- 6: Fluidkammer
- 7: Antriebselement
- 8: Zahnrad
- 9: Gehäuse
- 9a: Gehäusegewinde
- 10: Öffnung
- V: Vortriebsrichtung

## Patentansprüche

1. Ampulleneinheit für ein Verabreichungsgerät, insbesondere ein Injektionsgerät oder eine Injektionsspritze, wobei die Ampulleneinheit zur wiederholten reproduzierbaren Abgabe von kleinen Mengen eines flüssigen Medikaments ausgebildet ist, wobei das flüssige Medikament Insulin oder ein Hormonpräparat umfasst, umfassend
eine Ampulle (1) mit einem distalen Ende mit einer Öffnung (10) zur Abgabe eines fluiden Produkts, und einem proximalen Ende mit einer Öffnung zur Aufnahme einer Verdrängungskörpereinheit,
einen Adapter (4), der mit der Ampulle (1) derart durch Verklebung, Verschweissung oder andere Verbindungen verdreh- und verschiebesicher verbunden ist, derart dass gesichert ist, dass die Ampulle sich zum Adapter relativ weder in Längsrichtung noch in Umfangsrichtung bewegen kann, und
eine Kolbeneinheit (2, 3), welche die Verdrängungskörpereinheit bildet, wobei
der Adapter (4) mit einem Gewinde (4a) versehen ist, und an der Kolbeneinheit (2, 3) ein Gegengewinde (2a, 3a) zum Gewinde (4a) des Adapters (4) gebildet ist, das mit diesem in Gewindeeingriff ist,
**dadurch gekennzeichnet, dass**
die Kolbeneinheit (2, 3) mit einem Antrieb (7, 8) verbunden ist, der ausgebildet ist, die Kolbeneinheit (2, 3) um eine Drehachse in Drehung zu versetzen, wodurch sich die Kolbeneinheit (2, 3) aufgrund des Gewindeeingriffs zwischen dem Gewinde (4a) des Adapters (4) und dem Gegengewinde (2a, 3a) der Kolbeneinheit (2, 3) in die Ampulle schraubt und die Abgabe des fluiden Produkts durch die Öffnung (10) bewirkt.

2. Ampulleneinheit nach einem der vorgehenden Ansprüche, wobei die Gewindesteigung der Gewinde (4a) und Gegengewinde (2a; 3a) ≤ 2 mm/U ist.

3. Ampulleneineinheit nach einem der vorgehenden Ansprüche, wobei die Gewindesteigung im Bereich von 0,5 mm/U ≤ 2 mm/U liegt.

4. Ampulleneinheit nach einem der vorgehenden Ansprüche, wobei es sich bei dem Gewinde (4a) um ein Innengewinde und bei dem Gegengewinde (2a; 3a) um ein Aussengewinde handelt oder vice versa.

5. Ampulleneinheit nach einem der Ansprüche 1 bis 4, wobei eine Mutter, die fest mit dem proximalen Ende der Ampulle (1) verbunden ist, den Adapter (4) bildet.

6. Ampulleneinheit nach einem der Ansprüche 1 bis 4, wobei der Adapter (4) die Ampulle (1) wenigstens teilweise umgibt und unlösbar mit der Ampulle (1) verbunden ist.

7. Ampulleneinheit nach Anspruch 6, wobei der Adapter (4) auf die Ampulle (1) aufgespritzt oder aufgeschrumpft ist.

8. Ampulleneinheit nach einem der Ansprüche 1 bis 7, wobei ein Kolben (2) die Kolbeneinheit bildet.

9. Ampulleneinheit nach einem der Ansprüche 1 bis 7, wobei die Kolbeneinheit (2, 3) aus einem Kolben (2) und einer Kolbenstange (3) besteht und das Gegengewinde (2a; 3a) am Kolben (2) und/oder an der Kolbenstange (3) gebildet ist.

10. Ampulleneinheit nach Anspruch 9, **dadurch gekennzeichnet, dass** der Kolben (2) mit der Kolbenstange (3) derart verdrehsicher verbunden ist, dass der Kolben jede Drehbewegung der Kolbenstange mitmacht, mit der sich der Kolben in distaler Richtung in die Ampulle (1) hineinschraubt.

11. Ampulleneinheit nach Anspruch 9 oder 10, **dadurch gekennzeichnet, dass** die Aussenseite des Kolbens (2) konkav geformt ist, und der Kolben an seinem distalen und an seinem proximalen Ende je eine Dichtung (5) aufweist.

12. Ampulleneinheit nach einem der Ansprüche 9 bis 11, wobei der Kolben (2) mit der Kolbenstange (3) lösbar, beispielsweise über eine Schraub- oder Steckverbindung, verbunden ist.

13. Ampulleneinheit nach einem der Ansprüche 9 bis 11, wobei der Kolben (2) mit der Kolbenstange (3) unlösbar verbunden ist.

14. Ampulleneinheit nach Anspruch 13, wobei der Kolben (2) und die Kolbenstange (3) einstückig, vorzugsweise aus Kunststoff, gebildet sind.

15. Ampulleneinheit nach einem der vorgehenden Ansprüche, wobei der Kolben (2) oder die Kolbenstange (3) mit einem Antrieb, der den Kolben (2) und die Kolbenstange (3) rotativ antreibt, verbindbar sind.

## Claims

1. Ampoule unit for an administration device, in particular an injection device or an injection syringe, wherein the ampoule unit is configured for the repeated, reproducible dispensing of small quantities of a fluid medication, wherein the fluid medication includes insulin or a hormone product, comprising
an ampoule (1) with a distal end with an opening (10) for emitting a fluid product, and a proximal end with an opening for accommodating a displacement body unit, an adapter (4) that is connected by adhesion, welding or other connections to the ampoule (1) in a twist and slide-proof manner in such a way that it is ensured that the ampoule can move neither in the longitudinal direction nor in the circumferential direction relative to the adapter, and
a plunger unit (2, 3) which forms the displacement body unit,
wherein the adapter (4) is provided with a thread (4a), and on the plunger unit there is (2, 3) a counter-thread (2a, 3a) to the thread (4a) of the adapter (4) with which it is in threaded engagement,
**characterised in that**
the plunger unit (2, 3) is connected to an actuator (7, 8) which is configured to rotate the plunger unit (2, 3) about an axis of rotation, through which the plunger unit (2,3) due to the thread engagement between the thread (4a) of the adapter (4) and the counter-thread (2a, 3a) of the plunger unit (2, 3) screws itself into the ampoule and brings about emission of the fluid product through the opening (10).

2. Ampoule unit according to any one of the preceding claims wherein the thread pitch of the thread (4a) and the counter-thread (2a; 3a) is ≤ 2mm/rev.

3. Ampoule unit according to any one of the preceding claims wherein the thread pitch is in the range 0.5 mm/rev. ≤ 2 mm/rev.

4. Ampoule unit according to any one of the preceding claims wherein the thread (4a) is an internal thread and the counter-thread (2a; 3a) is an external thread or vice-versa.

5. Ampoule unit according to any one of claims 1 to 4 wherein a nut, which is firmly connected to the proximal end of the ampoule (1), forms the adapter (4).

6. Ampoule unit according to any one of claims 1 to 4 wherein the adapter (4) at least partially surrounds the ampoule (1) and is non-detachably connected with the ampoule (1).

7. Ampoule unit according to claim 6 wherein the adapter (4) is injected or shrunk onto the ampoule (1).

8. Ampoule unit according to any one of claims 1 to 7 wherein a plunger (2) forms the plunger unit.

9. Ampoule unit according to any one of claims 1 to 7 wherein the plunger unit (2, 3) comprises a plunger (2) and a plunger rod (3) and the counter-thread (2a; 3a) is formed on the plunger (2) and/or on the plunger rod (3).

10. Ampoule unit according to claim 9 **characterised in that** the plunger (2) is connected in a twist-proof manner with the plunger rod (3) in such a way that the plunger also undergoes every rotational movement with which the plunger screws itself in the ampoule (1) in the distal direction.

11. Ampoule unit according to claim 9 or 10 **characterised in that** the outer side of the plunger (2) is concave in shape and the plunger has seal (5) at its distal end as well as at its proximal end.

12. Ampoule unit according to any one of claims 9 to 11 wherein the plunger (2) is detachably connected to the plunger rod (3), for example by way of a screw or plug connection.

13. Ampoule unit according to any one of claims 9 to 11 wherein the piston (2) is non-detachably connected to the piston rod (3).

14. Ampoule unit according to claim 13 wherein the plunger (2) and the plunger rod (3) are formed in one piece, preferably made of plastic.

15. Ampoule unit according to any one of the preceding claims wherein the plunger (2) or the plunger rod (3) are connectable to an actuator, which drives the plunger (2) and the plunger rod (3) in a rotational manner.

## Revendications

1. Unité d'ampoule pour un appareil d'administration, notamment un appareil d'injection ou une seringue d'injection, l'unité d'ampoule étant constituée pour la délivrance répétée reproductible de petites quantités d'un médicament liquide, le médicament liquide comprenant l'insuline ou une préparation hormonale, comprenant
une ampoule (1) avec une extrémité distale avec une ouverture (10) pour délivrer un produit liquide et une extrémité proximale avec une ouverture pour recevoir une unité de corps de déplacement,
un adaptateur (4) qui est relié à l'ampoule (1) de façon solidaire en rotation et en déplacement par collage, soudage ou autres liaisons de telle sorte qu'il est assuré que l'ampoule ne pourra se déplacer relativement vers l'adaptateur ni en direction longitudinale ni en direction périphérique, et
une unité de piston (2,3), laquelle forme l'unité de corps de déplacement,
l'adaptateur (4) étant doté d'un filetage (4a) et un contre-filetage (2a,3a) étant formé sur l'unité de piston (2,3) pour le filetage (4a) de l'adaptateur (4), qui est en prise de filetage avec celle-ci, **caractérisée en ce que**
l'unité de piston (2,3) est reliée à un système d'entraînement (7,8) qui est constitué pour déplacer en rotation l'unité de piston (2,3) autour d'un axe de rotation, l'unité de piston (2,3) se vissant de ce fait dans l'ampoule en raison de la prise en filetage entre le filetage (4a) de l'adaptateur (4) et le contre-filetage (2a,3a) de l'unité de piston (2,3) et générant la délivrance du produit liquide par l'ouverture (10).

2. Unité d'ampoule selon l'une quelconque des revendications précédentes, le pas de filetage du filetage (4a) et du contre-filetage (2a,3a) étant ≤ 2mm/tr.

3. Unité d'ampoule selon l'une quelconque des revendications précédentes, le pas de filetage du filetage se situant dans une plage de 0,5 mm/tr ≤ 2mm/tr.

4. Unité d'ampoule selon l'une quelconque des revendications précédentes, s'agissant d'un filetage intérieur pour le filetage (4a) et d'un filetage extérieur pour le contre-filetage (2a,3a), ou vice versa.

5. Unité d'ampoule selon l'une quelconque des revendications 1 à 4, un écrou, relié fermement à l'extrémité proximale de l'ampoule (1), formant l'adaptateur (4).

6. Unité d'ampoule selon l'une quelconque des revendications 1 à 4, l'adaptateur (4) entourant au moins en partie l'ampoule (1) et étant relié de façon indissociable à l'ampoule (1).

7. Unité d'ampoule selon la revendication 6, l'adaptateur (4) étant projeté ou emmanché sur l'ampoule (1)

8. Unité d'ampoule selon l'une quelconque des revendications 1 à 7, un piston (2) formant l'unité de piston.

9. Unité d'ampoule selon l'une quelconque des revendications 1 à 7, l'unité de piston (2,3) étant composée d'un piston (2) et d'une tige de piston (3) et le contre-filetage (2a,3a) étant formé sur le piston (2) et/ou sur la tige de piston (3).

10. Unité d'ampoule selon la revendication 9, **caractérisée en ce que** le piston (2) est relié solidaire en rotation à la tige de piston (3) de telle sorte que le piston effectue conjointement chaque mouvement de rotation de la tige de piston avec laquelle le piston se visse intérieurement dans l'ampoule (1) en direction distale.

11. Unité d'ampoule selon la revendication 9 ou 10, **caractérisée en ce que** le côté extérieur du piston (2) est de forme concave et le piston comporte respectivement un joint d'étanchéité (5) à son extrémité distale et à son extrémité proximale.

12. Unité d'ampoule selon l'une quelconque des revendications 9 à 11, le piston (2) étant relié de façon dissociable à la tige de piston (3), par exemple par un raccord à vis ou enfichable.

13. Unité d'ampoule selon l'une quelconque des revendications 9 à 11, le piston (2) étant relié de façon indissociable à la tige de piston (3).

14. Unité d'ampoule selon la revendication 13, le piston (2) et la tige de piston (3) étant formés en une seule pièce, de préférence en matière plastique.

15. Unité d'ampoule selon l'une quelconque des revendications précédentes, le piston (2) ou la tige de piston (3) pouvant être reliés à un système d'entraînement qui entraîne de façon rotative le piston (2) et la tige de piston (3).
